# EUROPEAN PATENT APPLICATION

(11) **EP 2 011 534 A1**
(43) Date of publication of application: **07.01.2009**
(21) Application number: 07012988.7
(22) Date of filing: 03.07.2007
(51) Int. Cl.: A61M 15/00, B05B 1/00

(54) **Metered dose inhaler actuator**

(71) Applicant: CHIESI FARMACEUTICI S.p.A., 43100 Parma (IT)
(72) Inventor: Brambilla, Gaetano, 43100 Parma (IT); Church, Tanya Cathleen, 43100 Parma (IT); Lewis, David Andrew, 43100 Parma (IT); Meakin, Brian John, 43100 Parma (IT)
(74) Representative: Minoja, Fabrizio

(57) **Abstract**

A metered dose inhaler actuator for pressurised aerosol containing a formulation of at least one medicament in a liquefied propellant gas.
The actuator (2) comprises:
- a nozzle block (5) having a bore (11) to receive a valve stem (7),
- a sump (8) in connection with a bore (11), wherein the propellant formulation expands upon actuation of the inhaler,
- a nozzle channel (6), exiting from the sump (8) and aligned with a mouthpiece (4) wherein the sump (8) is characterised by an internal volume comprised between 12 and 2 mm³ to avoid deposits of the medicament during the delivery into the chamber and/or into the nozzle channel which could cause a reduction in the delivered dose and an increase in the occurrence of the clogging of the inhaler.
The actuator of the invention is particularly useful in the administration of pressurised metered dose inhaler formulations, in solution and/or in suspension, wherein the concentration of the active ingredient/s of the formulation is/are particularly high and in particular suitable to administer at least 100 μg/dose, preferably at least 200 μg/dose, even more preferably at least 400 μg/dose.

## Description

### FIELD OF THE INVENTION

The present invention relates to a metered dose inhaler actuator for pressurised aerosol containing a formulation of at least one medicament in a liquefied propellant gas.

The present invention also relates to the use of said metered dose inhaler to optimize the output characteristics of medicament formulations in solutions or in suspension in a liquefied gas propellant.

### BACKGROUND OF THE INVENTION

Among the many devices to deliver medicaments to the lung, metered dose inhalers (MDIs) are widely used.

MDIs are aerosol delivery systems designed to deliver a medicament formulated with a compressed, low boiling point liquid gas propellant. MDIs are designed to meter a predetermined quantity of the medicament, completely dissolved (in solution) or micronised and suspended in the formulation and dispense the dose as an inhalable aerosol cloud.

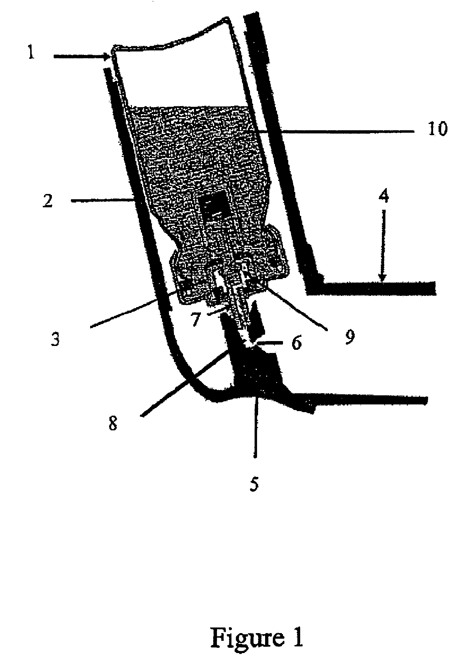

A typical commercially available MDI is shown in **Figure 1** and includes an actuator **2** in which a canister **1** is positioned. The canister **1** contains a liquid formulation **10** wherein the medicament is in solution or in suspension with a low boiling point propellant. The canister **1** is normally provided with a metering valve **3** for measuring discrete doses of the medicament formulation.

The metering valve **3** is fitted in a bore within a nozzle block **5** in the actuator **2.** The nozzle block **5** comprises a sump **8** in connection with the bore wherein the propellant formulation expands upon actuation of the inhaler and a nozzle channel **6** to deliver the metered dose.

Conventional pressurized metered dose inhaler actuators have variable nozzle channel diameters 6 from 0.25 to 0.45mm and lengths from 0.30 to 1.7mm, and sump volume ranging from 19 to 45 mm³.

A problem with known MDIs is that of adequately matching the dimensions of the nozzle channel length and diameter to the particular drug formulation and carrier-propellant. Different drugs have different flow and dispersion characteristics (particularly as between suspensions wherein drug particles are dispersed in the formulation and solutions wherein the drug is completely dissolved in the formulation) and it is often difficult to achieve the optimum balance between the plume shape, total dose volume and plume duration.

It has been disclosed (Lewis D.A. et al., Respiratory Drug Delivery VI, 363-364, 1998) that using commercially available actuators for delivering solution formulations of aerosol pressurized with HFA, the reduction in the nozzle channel diameter from 0.42 to 0.25 mm induces a desirable increase in the fine particle dose (FPD) of the aerosol produced.

Even if in general small nozzle channel diameters increase the FPD they may present various potential disadvantages. A small orifice is capable of restricting flow, which often causes increased material deposition, from the non-volatile components of the formulation, on the surfaces of the sump or of the nozzle channel of conventional devices. In turn the build-up or detachment of aggregated drug or non-volatile components of the formulation may potentially cause clogging of the nozzle channel, reducing or blocking the dose delivered to the patient.

Small orifices may also increase the spray angle of the plume, making it more disperse, and thus increasing deposition of the drug on the interior surfaces of the device (sump, nozzle channel and mouthpiece) and in the mouth of the patient. This unintended deposition tends to reduce the amount of drug delivered to the lung, increase the amount ingested, potentially contributing to cause side effects.

The unintended deposition problems become particularly important when the concentration of the active ingredient/s of the formulation is/are high and in particular suitable to administer at least 100 µg/dose.

A number of alternatives have been proposed in the prior art to solve these disadvantages.

EP 373753, for example, suggests redesigning the exit orifice to include a spout to prevent cumulative drug build-up on the nozzle.

The sump and the nozzle channel geometry and actuator internal geometry have also been reported to be of significance with regard to reducing the risk of actuator clogging. WO 03/002169 in particular proposed internal expansion chamber (sump) designs that ensure a smooth, rounded, interior surface and promote a continuous flow path towards the spray orifice to improve drug delivery consistency and reduce actuator blockage.

WO 01/58508 suggests that the deposition within the nozzle block may be reduced by incorporating a smaller expansion chamber volume. However the expansion chamber volume is defined as the sum of the internal void of the valve stem conduit and the actuator's internal chamber volume. Moreover such reduction in volume is associated with a complex design of the internal chamber which has to have a tubular, smooth-sided configuration that non-abruptly curves from the inlet to the outlet of the chamber so to reduce fluid resistance within the internal chamber, thereby reducing deposition.

The drawback of such a device is that each component as to be optimised for dimension and shape.

It has now been found that by reducing the sump volume within the nozzle block in the metered dose inhaler actuator it is possible to reduce deposition of the active ingredient and/or of the low volatility components present in the formulation in the device and avoid device failure due to clogging even when, to generate high fine particle fractions, the nozzle channel features a diameter smaller than the standard.

### SUMMARY OF THE INVENTION

The present invention is directed to an actuator 2 for a metered dose inhaler, comprising:
- a nozzle block 5 having a bore 11 suitable to receive a valve stem 7,
- a sump 8 in connection with the bore 11 wherein when in use a propellant formulation expands upon actuation of the metered dose inhaler,
- a nozzle channel 6, exiting from the sump 8 and aligned with a mouthpiece 4 characterized in that the sump 8 has an internal volume smaller than 12 and bigger than 2 mm³.

According to an embodiment of the present invention the sump 8 has a volume smaller than 9 and bigger than 3 mm³, preferably smaller than 7 and bigger than 5 mm³, even more preferably equal to about 6.

According to an embodiment of the present invention the bore 11 has a size suitable to receive a valve stem 7 having an internal volume comprised between 15 and 150 mm³, preferably comprised between 25 and 100 mm³ even more preferably comprised between 50 and 90 mm³ and most preferably comprised between 70 and 75 mm³.

According to an embodiment of the present invention the nozzle channel has a diameter smaller than 0.25 mm and a channel length comprised between 0.7 and 0.4 mm, preferably a diameter equal to 0.22 mm and channel length equal to 0.65 or 0.45 mm.

The present invention is also directed to a metered dose inhaler comprising:
- an actuator 2 comprising a nozzle block 5 having a bore 11 suitable to receive a valve stem 7, a sump 8 in connection with the bore 11, a nozzle channel 6, exiting from the sump 8 and aligned with a mouthpiece 4
- a canister 1 closed by
- a metering valve comprising a valve stem **7** to be fitted in the bore **11** within the nozzle block **5** in the actuator **2**
characterized in that the sump **8** has an internal volume smaller than **12** and bigger than **2** mm³.

Preferably the canister **1** in the metered dose inhaler is filled with an aerosol formulation comprising at least one active ingredient, a co-solvent, an HFA propellant. Even more preferably the total concentration of the at least one active ingredient of the formulation is suitable to administer at least 100 µg/dose, preferably at least 200 µg/dose, even more preferably at least 400 µg/dose.

Preferably the metering valve has a metering chamber of 100, 63, 50 µl, preferably of 63 µl.

According to an embodiment of the present invention the aerosol formulation comprises budesonide, preferably comprises budesonide and carmoterol.

Further the invention is directed to the use of an actuator comprising a sump **8** having an internal volume smaller than **12** and bigger than **2** mm³to prevent the clogging of the actuator when used in a metered dose inhaled filled with an aerosol formulation.

### DESCRIPTION OF THE FIGURES

**Figure 1** shows a metered dose inhaler commonly used.
**Figure 2** shows a preferred design of an actuator of the present invention.
**Figure 3** shows an enlargement of the nozzle block within a preferred design of an actuator of the present invention.

### DETAILED DESCRIPTION OF THE INVENTION

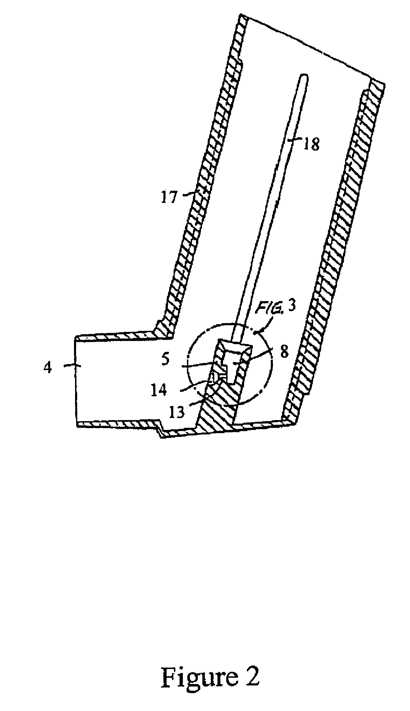

A typical commercially available MDI is shown in **Figure 1** and includes an actuator **2** in which a canister **1** is optionally positioned on supporting means 18 (only shown in Figure2). The canister 1 contains a liquid formulation 10 wherein the medicament is in solution and/or in suspension with a low boiling point propellant and optionally with one or more pharmaceutically acceptable additive and/or excipient.

The canister is normally provided with a metering valve 3 for measuring discrete doses of the medicament formulation fluid per actuation. The metering valve comprises a metering chamber 9 and a valve stem 7 to be fitted in a bore 11 within a nozzle block 5 in the actuator 2.

The actuator 2 comprises:
a nozzle block 5 having a bore 11 suitable to receive a valve stem 7 which acts as a conduit to deliver the metered dose,
a sump 8 in connection with the bore 11 wherein, when in use, the propellant formulation expands upon actuation of the inhaler, a nozzle channel 6, exiting from the sump 8 and aligned with a mouthpiece 4.

For the administration of a medicament through a MDI the patient places the mouthpiece 4 against his lips and actuates the MDI by depressing the canister into the actuator. Upon actuation a metered dose, measured by the valve, is expelled from the valve stem. The expelled dose passes through the internal expansion chamber 8 of the nozzle block 5 and exits from the nozzle channel 6. The patient starts the inhalation through the mouthpiece upon the release of the metered dose following the actuation of the inhaler.

Conventional pressurized metered dose inhaler actuators have variable nozzle channel diameters from 0.25 to 0.42 mm and a length from 0.30 to 1.7 mm, and sump volume ranging from 19 to 45 mm³.

It has now been found that by reducing the sump volume it is possible to reduce deposition of the active ingredient/s and/or of the low volatility components present in the formulation in the device and avoid device failure due to clogging even when, to generate high fine particle fractions, the nozzle channel features a diameter lower than 0.25 mm and a length lower than 0.7 mm.

**Figure 2** shows a preferred design of an actuator **2** according to one embodiment of the present invention.

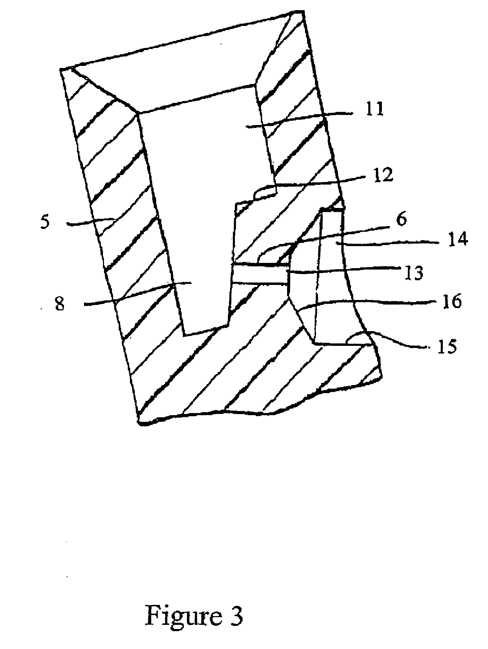

**Figure 3** shows an enlargement of a nozzle block **5** within a preferred design of an actuator **2** according to one embodiment of the present invention.

The nozzle block **5** has a bore **11** suitable to receive the valve stem **7** of the canister (not shown). The bore **11** in its lower part has a step **12** to bearing the valve stem when it is present.

According to another embodiment the bore **11** has two or more steps **12** to bear the valve stem **7** and according to a further embodiment the step **12** extends all around the lower part of the bore **11.**

In general, the bore **11** has to have means **12** to bear the valve stem **7** in its correct position when the valve is fitted thereupon. A person skilled in the art will recognize that the shape of such means **12** may vary and may be adapted to the specific MDI.

The dimension of the bore **11** may vary according to the dimension of the valve stem **7.** The valve stem may have any internal volume compatible with the MDI size. It has in fact been found that the fine particle dose of the delivered medicament is independent from the internal volume of the valve stem.

However preferably the valve stem **7** has an internal volume comprised between 15 and 150 mm³, preferably comprised between 25 and 100 mm³ even more preferably comprised between 50 and 90 mm³ and most preferably comprised between 70 and 75 mm³.

The metering valve has a metering chamber preferably of 100, 63, 50 µl, even more preferably of 63 µl.

**Figure 3** shows a sump **8** in connection with the bore **11** wherein, when in use the propellant formulation expands upon actuation of the inhaler. The shape of the sump **8** is conventional, having angles and corners.

Although it is more convenient to use a conventional sump **8,** according to other embodiments the sump **8** may be redesigned to have a rounded corner free shape, for example it may have a U-shape.

According to the present invention the sump **8** volume is lower than **12** mm³ and bigger than 2 mm³. Preferably the sump volume is lower than 9 mm³ and bigger than 3 mm³. Even more preferably the sump volume is lower than 7 mm³ and bigger than 5 mm³. According to another embodiment of the present invention the sump volume is about 6 mm³. According to a further embodiment the sump volume is about 5 mm³. According to a further embodiment the sump volume is about 7 mm³.

As used herewith the term "about" encompasses dimensions, which differ of less than 2% from the dimension mentioned.

The sump **8** is connected to a nozzle channel **6** wherein the formulation after been expanded flows to reach the mouthpiece **4.**

The nozzle channel **6** ends in an aperture **13** positioned in a cylindrical recess **14** having a parallel sided portion **15** and a frusto-conical base **16.**

The nozzle channel may have diameters ranging from 0.15 to 0.4 mm and a length from 0.30 to 1.7 mm.

However in order to increase the fine particle dose the channel diameter is preferably smaller than 0.25 mm and has a length comprised between 0.4 and 0.7 mm. Even more preferably the channel diameter is equal to 0.22 mm and the channel length is 0.65 mm or 0.45 mm or any length in between.

For certain formulations it would be useful to utilize laser-drilled actuator orifices having a diameter ranging from 0.10 to 0.22 mm, in particular from 0.12 to 0.18 mm as those described in WO 03/053501.

Although it is more convenient to use conventional nozzle channel, and aperture shapes, according to other embodiments they may be redesigned to have alternative shapes. According to another embodiment more than **1** nozzle channel **6** and/or aperture **13** may be present.

In order for a patient to insert the mouthpiece **4** at the correct orientation for discharge of the spray whilst at the same time holding the body portion **17** of the actuator and the canister **1** at a convenient angle, the longitudinal axis of the mouthpiece **4** is preferably inclined at an angle of about 105 degrees with respect to the longitudinal axis of the body portion **17** of the actuator **2** and of the nozzle block **5** as shown in **Figure 2****.**

In another aspect the present invention provides a metered dose inhaler comprising the actuator of the present invention, a canister closed by means of a metering valve, said canister being filled with an aerosol formulation in a liquefied gas propellant.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto a can to form an empty canister. The aerosol formulation is pressure filled through the metering valve into the canister.

In an alternative process, the aerosol formulation is added to an open canister under conditions which are sufficiently cold that the formulation does not vaporize, and then a metering valve crimped onto the canister.

In an alternative process, a medicament dissolved in the solubilising agent is dispensed into an empty canister, a metering valve is crimped thereon, and then the propellant is filled into the canister through the valve. Preferably, the processes are carried out in inert atmosphere, for instance by insufflating nitrogen, in order to avoid the uptake of humidity from the air.

Each filled canister is conveniently fitted into the actuator of the present invention through which upon actuation a medicament may be delivered from the filled canister via the metering valve to the mouth of a patient.

Suitable canisters generally are capable of withstanding the vapour pressure of the propellant. The canister may be of any material such as glass, plastic or plastic-coated glass or preferably metal.

More preferably, the formulations will be filled in canisters having part of all of the internal surfaces made of aluminium, anodized aluminium, and stainless steel. If the formulation so requires, for chemical and/or physical stability problems, the canister, preferably an aluminium canister, may be lined with an inert organic coating. Examples of preferred coatings are epoxy-phenol resins, perfluorinated polymers such as perfluoroalkoxyalkane, perfluoroalkoxyalkylene, perfluoroalkylenes such as poly-tetrafluoroethylene, fluorinated-ethylene-propylene, polyether sulfone and/or mixture thereof. Other suitable coatings may be polyamide, polyimide, polyamideimide, polyphenylene sulfide or combinations thereof.

The metering valve comprises a metering chamber and it is designed to deliver a metered amount of the formulation per actuation and incorporates valve seals to prevent leakage of propellant through the valve. The valve seals will preferably be manufactured from a material which is inert to the formulation. They may comprise any suitable elastomeric material such as for example low density polyethylene, chlorobutyl, black and white butadiene-acrylonitrile rubbers, butyl rubber and neoprene.

Thermoplastic elastomer valves as described in WO 92/11190 and valves containing EPDM rubber are especially suitable. Suitable valves are commercially available from manufacturers well known in the aerosol industry, for example, from Valois, France (e.g. DF10, DF30, DF31, DF60), Bespak plc UK (e.g. BK300, BK356, BK357) and 3M-Neotechnic Ltd. UK (e.g. SpraymiserTM).

The valve, especially the metering chamber, will preferably be manufactured of a material which is inert to the formulation. Particularly suitable materials for use in manufacture of the metering chamber include polyesters e.g. polybutyleneterephthalate (PBT) and acetals, especially PBT.

Materials of manufacture of the metering chamber and/or the valve stem may desirably be fluorinated, partially fluorinated or impregnated with fluorine containing substances in order to resist drug deposition.

The formulation may be a solution or a suspension of at least one suitable medicament in a liquefied gas propellant. The aerosol solution formulations offer the advantage of being homogeneous being the active ingredient which completely dissolved in the propellant vehicle or in the mixtures thereof with suitable co-solvents. Solution formulations also obviate physical stability problems associated with suspension formulations, thus assuring reproducible dosage.

The propellant is preferably, 1,1,1,2-tetrafluoroethane (HFA 134a) or 1,1,1,2,3,3,3-heptafluoropropane (HFA 227) or a mixture thereof. In alternative propellants such as carbon dioxide or other which are gaseous at room temperature and standard atmospheric pressure may be used.

The preferred co-solvents are lower alkyl (C₁-C₄) alcohols, polyols, polyalkylene glycols and their combinations For example the co-solvent may be ethanol, propanol, propylene glycol, polyethylene glycol, glycerol and/or their mixture. Ethanol is particularly preferred.

According to a preferred embodiment, the aerosol formulation which may be used with the actuator of the present invention comprises a medicament, a HFA propellant, an amount of ethanol up to 30%, preferably up to 20%, more preferably up to 15% w/w.

Optionally a low volatility component may be added to the formulation. The low volatility component preferably has a vapour pressure at 25°C lower than 0.1 kPa, more preferably lower than 0.05 kPa. Examples of suitable low volatility components are glycols, particularly propylene glycol, polyethylene glycol and glycerol, esters for example ascorbyl palmitate, isopropyl myristate and tocopherol esters.

The formulation may contain from 0.2 to 10% w/w of said low volatility component, preferably between 0.5 and 2.0% w/w.

The aerosol formulation may further comprise an additional co-solvent having higher polarity than the co-solvent, allowing reducing the co-solvent amount and thereby modulating the particle size of the produced aerosol droplets.

If the cosolvent is ethanol, the additional co-solvents with a higher polarity may be a lower alkyl (C₁-C₄) alcohol, a polyols or a polyalkylene glycol.

The preferred polyols include propylene glycol and glycerol and the preferred polyalkylene glycol is polyethylene glycol.

Among the co-solvents with a higher polarity than ethanol water is to be considered comprised.

Preferably the additional co-solvent is added in amount from 0.2% to 10% w/w, preferably from 0.5 to 10% w/w, more preferably from 0.5 to 6% w/w, even more preferably from 1 to 2% w/w.

The ratio between the co-solvent and the additional co-solvent is a critical factor for an efficient aerosolization. The selection of said ratios may be anyhow made by the skilled in the art on the basis of the chemical-physical characteristics of the considered active ingredient/s.

Advantageously an organic or inorganic acid may be added to the solution. Preferably the acid is a mineral acid, more preferably the acid is a strong mineral acids such as hydrochloric, nitric or phosphoric acid.

In certain cases, formulation may optionally contain small amounts of additional components such as surfactants or other additives which are preservatives, buffers, antioxidants, radical quenchers, sweeteners and taste masking agents.

Active ingredients which may be used in the aerosol formulation are long-acting β₂-adrenergic agonists (LABAs) such as formoterol, salmeterol, carmoterol indacaterol, stereoisomers, salts and solvates thereof.

The active ingredient may be a long acting β2-agonists belonging to the formula sketched below 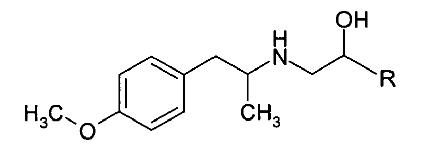 wherein R is more preferably 1-formylamino-2-hydroxy-phen-5-yl (formoterol) or 8-hydroxy-2(1H)-quinolinon-5-yl (carmoterol) or one of their corresponding stereoisomers or salts.

The active ingredient may also be a steroid such as budesonide and its 22R-epimer, beclometasone dipropionate (BDP), triamcinolone acetonide, fluticasone propionate, fluticasone furoate, flunisolide, mometasone furoate, rofleponide, ciclesonide.

Alternatively the active ingredient may be antimuscarinic or anticholinergic atropine-like derivative such as ipratropium bromide, oxytropium bromide, tiotropium bromide, glycopyrrolate bromide, revatropate, or the compounds disclosed in WO 03/053966.

Alternatively the active ingredient may be any medicament useful for the management of respiratory diseases such as methylxanthines, anti-eukotrienes and phosphodiesterase inhibitors (PDE) inhibitors and in particular PDE4 inhibitors such as roflumilast or cilomilast.

The active ingredient may be also any combination of the aforementioned active ingredient. The preferred combinations are carmoterol-budesonide, formoterol-BDP and in general combination comprising a ß₂-agonist.

The concentration of the active ingredient in the HFA formulation will depend on the therapeutic amount to be delivered preferably in one or two actuations.

In general, the actuator of the invention is particularly useful in the administration of pressurised metered dose inhaler formulations, in solution and/or in suspension, wherein the total concentration of the at least one active ingredient of the formulation is suitable to administer at least 100 µg/dose, preferably at least 200 µg/dose, even more preferably at least 400 µg/dose.

It may be appreciated that by varying the metering chamber volume the concentration of the active ingredient has to vary accordingly to deliver the same dosage.

According to an embodiment of the present invention the aerosol formulation comprises budesonide, preferably comprises budesonide and carmoterol.

The aerosol formulation may be indicated for the treatment of mild, moderate or severe acute or chronic symptoms or for prophylactic treatment of respiratory diseases such as asthma and chronic obstructive pulmonary disease (COPD). Other respiratory disorders characterized by obstruction of the peripheral airways as a result of inflammation and presence of mucus such as chronic obstructive bronchiolitis and chronic bronchitis can also benefit by this kind of formulation.

Further the invention is directed to the use of an actuator comprising a sump 8 having an internal volume smaller than 12 and bigger than 2 mm³to prevent the clogging of the actuator when used in a metered dose inhaled filled with an aerosol formulation as described above.

The invention is better illustrated by the following example.

### EXAMPLE

A patient simulation has been performed upon a budesonide-carmoterol HFA MDI solution. Consistent drug delivery performance was demonstrated in through life testing with a standard actuator with an exit orifice of 0.22 mm.

The drug delivery performance during a patient simulation has been evaluated for four alternative actuator designs. Drug delivery performance has been presented for Budesonide only.

The actuators were actuated twice per day with a dosing interval of at least four hours.

The aerodynamic particle size distribution and delivered dose of each pMDI was determined at the beginning and end of life using Andersen Cascade Impactor (ACI) according to the procedure described in European Pharmacopoeia 2nd edition, 1995, part V.5.9.1, pages 15-17.

The Shot Weight and Delivered Dose and their variance were measured using the Dosage Unit Sampling Apparatus (DUSA) as described in the European Pharmacopoeia 3rd edition, Supplement 2000, pages 1351-354. Shot weights were recorded throughout the study to evaluate through life metering performance of each pMDI.

Deposition of budesonide on each ACI plate is determined by high performance liquid chromatography (HPLC).

A sequence of 4 cumulative doses was fired into the ACI for each measurement and all shot weights were recorded.

### Budesonide-carmoterol HFA MDI solution

Budesonide-carmoterol MDI solution formulations in HFA 134a contain 15% w/w ethanol and 0.002% w/w phosphoric acid (15M), presented in a FEP-PES coated aluminium cans fitted with 63 µl valves.

Each MDI contains 60 (plus 40 overage) doses, delivering 1 g of carmoterol and 200 µg of budesonide per puff.

### ACTUATORS

This example presents data from four actuator designs A, B, C & D. Detail of each design is summarised in Table 1. Actuator A has an orifice diameter = 0.22 mm; an orifice length = 0.65 mm; a sump volume = 19.66 mm³. Compared to Actuator A, Actuators B, C and D have reduced sump geometries. Actuators C & D were designed to have a sump volume of 6.07 mm³. In addition, Actuator D has a reduced exit orifice length (0.45 mm) compared to Actuators A, B and C (0.65 mm).

Prior to the study the surface finish of all actuators was evaluated by microscopy to ensure no effects of worn tooling would perturb the performance observations from each of the actuator designs.

**Table 1: Summary of Actuator designs A, B, C and D.**

| **Actuator ID** | **Exit Orifice (mm)** | | |
|---|---|---|---|
| | **Diameter** | **Length** | **Sump Volume (mm³)** |
| A | 0.22 | 0.65 | 19.66 |
| B | | | 12.37 |
| C | | | 6.07 |
| D | | 0.45 | |

### DELIVERED DOSE

Table 2 presents beginning of can life delivered dose data for actuator designs A, B, C & D. The mean delivered dose (± SD) obtained for actuator design A, B, C and D was 173 ± 7 mg, 173 ± 10 mg, 167 ± 8 mg and 174 ± 7 mg respectively. Mean shot weights ranged from 70 mg to 73 mg.

**Table 2: Beginning of Can Life Delivered Dose Data - Actuator Design A, B, C & D.**

| **Actuator Design:** | **A** | | **B** | | **C** | | **D** | |
|---|---|---|---|---|---|---|---|---|
| **Budesonide Deposition per Actuation (µg)** | | | | | | | | |
| **Can ID:** | 1 | 2 | 1 | 2 | 1 | 2 | 1 | 2 |
| | 178 | 166 | 175 | 158 | 154 | 161 | 164 | 175 |
| | 164 | 167 | 180 | 164 | 173 | 177 | 180 | 183 |
| | 180 | 178 | 182 | 165 | 173 | 169 | 177 | 177 |
| | 180 | 173 | 186 | 173 | 170 | 160 | 168 | 166 |
| **Mean** | 173 | | 173 | | 167 | | 174 | |
| **Standard Deviation** | 7 | | 10 | | 8 | | 7 | |

| **Shot Weight (mg)** | | | | | | | | |
|---|---|---|---|---|---|---|---|---|
| **Mean** | 71.8 | 70.6 | 73.0 | 69.5 | 69.6 | 72.2 | 71.0 | 69.9 |
| **Standard Deviation** | 1.6 | 1.2 | 0.2 | 0.8 | 0.6 | 0.2 | 0.4 | 0.3 |

### PARTICLE SIZE CHARACTHERIZATION: ACI DATA

Table 3 presents beginning of can life ACI deposition and dose summary data. Consistent metered dose (199 ± 4.7 µg), delivered dose (183 ± 3.4µg), mass median aerodynamic diameter, MMAD (1.6 µm) and geometric standard deviation, GSD (2.0 - 2.4) was observed for all measurements.

Mean (n = 2) fine particle dose, FPD (≤5µm aerodynamic diameter), was determined to be 97, 98, 90 & 90 µg for actuator designs A-D respectively.

**Table 3: Dose Summary Data - Actuator Design A, B, C & D.**

| | | | | | | | | |
|---|---|---|---|---|---|---|---|---|
| **Actuator Design:** | **A** | | **B** | | **C** | | **D** | |

| **Dose Summary** | | | | | | | | |
|---|---|---|---|---|---|---|---|---|
| **Metered (µg)** | 209 | 202 | 196 | 199 | 196 | 195 | 196 | 198 |
| **Delivered (µg)** | 191 | 184 | 181 | 182 | 180 | 183 | 182 | 183 |
| **FPD ≤ 5 µm (µg)** | 101 | 93 | 96 | 100 | 88 | 92 | 87 | 92 |
| **MMAD (µm)** | 1.6 | 1.6 | 1.6 | 1.6 | 1.6 | 1.6 | 1.6 | 1.6 |
| **GSD** | 2.1 | 2.2 | 2.1 | 2.0 | 2.1 | 2.2 | 2.4 | 2.3 |

### THROUGH CAN LIFE SHOT WEIGH MEASUREMENTS

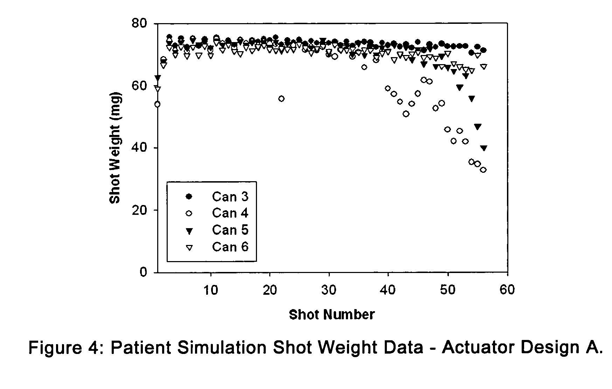

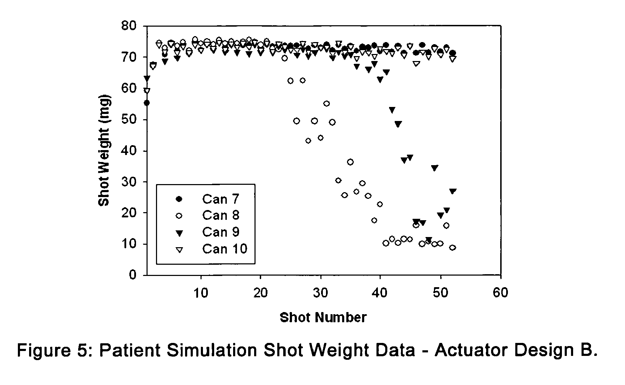

Shot weight data from the through life patient simulation is presented in figures 4 - 7 for actuator designs A - D respectively. As the patient simulation progressed, fluctuation in shot weight was observed for actuator designs A (cans 4 & 5, see figure 4) and B (cans 8 & 9, see figure 5). Shot weights of 33 - 40 mg on cans 4 and 5 (design A) and 9 - 11 mg on cans 8 and 9 (design B) are indicative of actuator blockage issues.

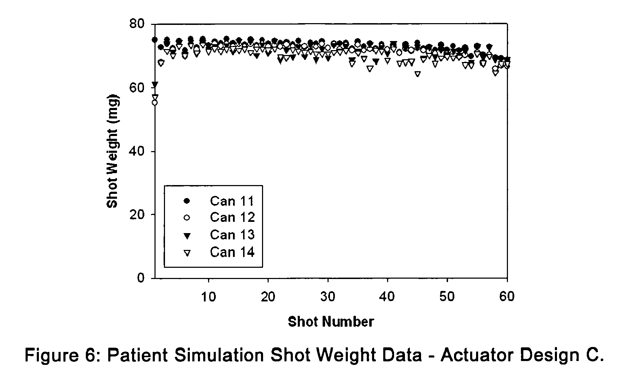

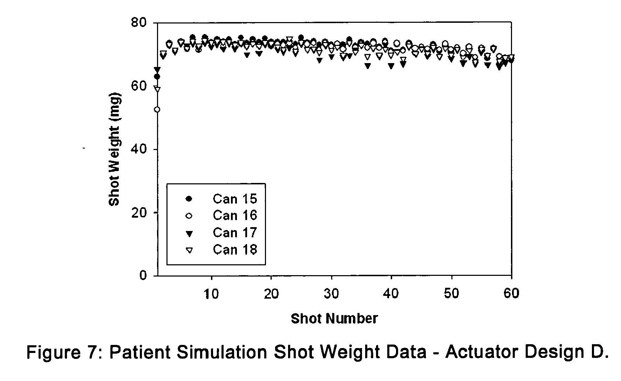

Consistent through can-life patient simulation shot weight data was observed for actuator design C and D (see figures 6 and 7). The mean shot weight (± standard deviation) for design A and B was 69.7 ± 7.5 mg and 63.9 ± 19.1 mg respectively. The lowest observed shot weight was consistent for design C (64.5 - 68.6 mg, n = 4) and design D (66.0 - 68.6 mg, n = 4), indicating no trend with regard to orifice length.

### PARTICLE SIZE CHARACTHERIZATION: ACI DATA

End of patient simulation ACI data was not collected for actuator design A & B due to undesirable blockage issues identified from the delivered dose data.

**Table 4: End of Patient Simulation ACI Dose Summary Data - Actuator Design C & D.**

| **Actuator Design:** | **C** | | | | **D** | | | |
|---|---|---|---|---|---|---|---|---|
| **Dose Summary** | | | | | | | | |
| **Delivered (µg)** | 181 | 174 | 177 | 174 | 177 | 177 | 171 | 183 |
| **FPD ≤ 5 µm (µg)** | 87 | 86 | 90 | 82 | 87 | 89 | 87 | 91 |
| **MMAD (µm)** | 1.7 | 1.7 | 1.7 | 1.7 | 1.6 | 1.5 | 1.6 | 1.7 |
| **GSD** | 2.3 | 2.3 | 2.2 | 2.3 | 2.3 | 2.2 | 2.2 | 2.4 |

In conclusion, no incidences of actuator blockage were observed for either of the actuators with a 6.07 µl sump volume C and D. Consistent shot weights were recorded throughout the patient simulation for both actuators. End of patient simulation delivered dose values were 185 ± 7 µg (sump volume 6.07 µl, exit orifice length = 0.65 mm) and 181 ± 3 µg (sump volume 6.07 µl, exit orifice length = 0.45 mm) and were comparable to those obtained at the beginning of can life (167 ± 8µg and 174 ± 7 µg respectively). ACI data obtained at the end of the patient simulation for the 6.07 µl sump designs was compared to that obtained at the beginning of can life. The average (±SD) results obtained for the two respective actuators (exit orifice length = 0.65 and 0.45 mm) were delivered dose: 178 ± 4 µg & 180 ± 3 µg, FPD: 88 ± 3 µg & 89 ± 2 µg, MMAD: 1.7 ± 0.1 µm & 1.6 ± 0.1 µm, and GSD: 2.2 ± 0.1 & 2.3 ± 0.1.

## Claims

1. An actuator **2** for a metered dose inhaler, comprising:
- a nozzle block **5** having a bore **11** suitable to receive a valve stem **7,**
- a sump 8 in connection with the bore wherein when in use a propellant formulation expands upon actuation of the metered dose inhaler,
- a nozzle channel **6,** exiting from the sump **8** and aligned with a mouthpiece **4 characterized in that** the sump **8** has an internal volume smaller than 12 and bigger than 2 mm³.

2. An actuator of claim **1** wherein the sump **8** has a volume smaller than **9** and bigger than 3 mm³.

3. An actuator of claim **2** wherein the sump **8** has a volume comprised between 5 and 7 mm³.

4. An actuator of claim **3** wherein the sump **8** has a volume of about **6** mm³.

5. An actuator of any of the preceding claims wherein the bore **11** has a size suitable to receive a valve stem **7** having an internal volume comprised between **15** and 150 mm³.

6. An actuator of claim **5** wherein the bore has a size suitable to receive a valve stem **7** having an internal volume comprised between 25 and 100 mm³.

7. An actuator of claim 5 wherein the bore has a size suitable to receive a valve stem **7** having an internal volume comprised between 70 and 75 mm³.

8. An actuator of any of the preceding claims wherein the nozzle channel has a diameter smaller than 0.25 mm a channel length comprised between 0.7 and 0.4 mm.

9. An actuator of claim 8 wherein the nozzle channel has a diameter equal to 0.22 mm and channel length equal to 0.65 and 0.45 mm.

10. A metered dose inhaler comprising, the actuator 2 according to any of the preceding claims and
- a canister 1 closed by
- a metering valve comprising a valve stem **7** to be fitted in the bore **11** within the nozzle block **5** in the actuator **2.**

11. A metered dose inhaler of claim 10 wherein the canister is filled with an aerosol formulation comprising at least one active ingredient, a co-solvent, an HFA propellant.

12. A metered dose inhaler of claim **11** wherein the total concentration of the at least one active ingredient of the formulation higher the at least one active ingredient of the formulation is suitable to administer at least 100 µg/dose, preferably at least 200 µg/dose, even more preferably at least 400 µg/dose.

13. A metered dose inhaler of claim **12** wherein the metering valve has a metering chamber of 63 µl.

14. A metered dose inhaler of claims **12** and **13** wherein the aerosol formulation comprises budesonide.

15. A metered dose inhaler of claim **14** wherein the aerosol formulation comprises budesonide and carmoterol.

16. The use of an actuator **2** comprising a sump **8** having an internal volume smaller than **12** and bigger than 2 mm³ to prevent the clogging of the actuator when used in a metered dose inhaled filled with an aerosol formulation.
